# EUROPÄISCHE PATENTANMELDUNG

(11) **EP 2 359 792 A2**
(43) Veröffentlichungstag der Anmeldung: **24.08.2011**
(21) Anmeldenummer: 11150183.9
(22) Anmeldetag: 05.01.2011
(51) Int. Cl.: A61G 7/08, H02K 41/00

(54) **Führungssystem für medizinische Anlagen, medizinische Anlage, fahrbare Transportvorrichtung sowie Verfahren**

(30) Priorität: 15.02.2010 DE 102010008014
(71) Anmelder: Siemens Aktiengesellschaft, 80333 München (DE)
(72) Erfinder: Weber, Ulrich, 61381, Friedrichsdorf (DE); Grübling, Peter, 35096, Weimar/Lahn (DE); Herrmann, Klaus, 90403, Nürnberg (DE)

(57) **Zusammenfassung**

Die Erfindung betrifft ein Führungssystem für medizinische Anlagen, aufweisend zumindest ein Bodenelement, welches sich entlang eines Weges der medizinischen Anlage erstreckt und in einem Bodenbereich des Weges angeordnet ist, und welches zur magnetischen Wechselwirkung derart ausgebildet ist, dass durch die magnetische Wechselwirkung des Bodenelements mit einem magnetischen Führungselement, das an einer fahrbaren Transportvorrichtung angeordnet ist, eine magnetische Anziehungskraft erzeugbar ist, wodurch eine Führung der fahrbaren Transportvorrichtung entlang des Bodenelements während eines Vorschubs der fahrbaren Transportvorrichtung entlang des Weges durch die medizinische Anlage erzielbar ist. Weiterhin betrifft die Erfindung eine fahrbare Transportvorrichtung, die mit dem magnetischen Führungselement in analoger Weise wechselwirkt, eine medizinische Anlage mit einem derartigen Führungselement und ein entsprechendes Verfahren zum Lenken der fahrbaren Transportvorrichtung entlang des Führungssystems.

## Beschreibung

Die Erfindung betrifft ein Führungssystem für medizinische Anlagen, wodurch eine fahrbare Transportvorrichtung, z.B. eine Patiententransportvorrichtung, gelenkt werden kann, sowie eine fahrbare Transportvorrichtung. Eine derartige Transportvorrichtung wird auch als "Trolley" bezeichnet. Weiterhin betrifft die Erfindung eine medizinische Anlage mit einer derartigen Führung sowie ein Verfahren zum Lenken einer derartigen Transportvorrichtung.

In einer medizinischen Anlage wie z.B. Kliniken müssen Patienten, die auf einem Trolley gelagert sind, oftmals über weite Strecken von einem Raum zu einem nächsten Raum gefahren werden, beispielsweise um bestimmte Untersuchungen/Therapien durchzuführen, die nur in bestimmten Räumen möglich sind.

Ein Beispiel für eine derartige medizinische Anlage ist eine Partikeltherapieanlage. Die Partikeltherapie ist dabei ein etabliertes Verfahren zur Behandlung insbesondere von Tumorerkrankungen. Da eine Partikeltherapieanlage vergleichsweise teuer ist, ist es notwendig, eine Partikeltherapieanlage effizient zu betreiben. Um dies zu ermöglichen, ist es bekannt, einen Patienten in einem Behandlungsraum lediglich zu bestrahlen und möglichst wenig Vorbereitung/Nachbereitung am Patienten in dem Behandlungsraum selbst vorzunehmen. Zu einer Vorbereitung gehört beispielsweise die Immobilisation eines Patienten. Die Vorbereitung/Nachbereitung findet daher oftmals in einem separaten Raum statt.

Zwischen dem Vorbereitungsraum und dem Behandlungsraum müssen mitunter beträchtliche Wege, die aus strahlenschutztechnischen Gründen oftmals labyrinthartig verwinkelt sind, zurückgelegt werden. Der Patient muss also zwischen beiden Räumen hin und her gefahren.

Es sind Lösungen bekannt, bei denen über ein optisches System die Fahrt einer Patientenliege überwacht wird; die Patientenliege wird über einen integrierten Steuermechanismus automatisch gelenkt.

Es ist die Aufgabe der Erfindung, ein Führungssystem für medizinische Anlagen bereitzustellen, das es ermöglicht, eine fahrbare Transportvorrichtung gut steuerbar und Kraft sparend zu fahren. Weiterhin ist es die Aufgabe der Erfindung, eine fahrbare Transportvorrichtung anzugeben, die entlang des Führungssystems gut steuerbar und Kraft sparend gefahren werden kann. Weiterhin ist es die Aufgabe der Erfindung, eine medizinische Anlage mit einem derartigen Führungssystem und ein Verfahren zum Lenken einer fahrbaren Transportvorrichtung anzugeben, das ein einfaches, sicheres und Kraft sparendes Lenken erlaubt.

Die Aufgabe wird gelöst durch ein Führungssystem nach Anspruch 1, durch eine medizinische Anlage nach Anspruch 6, durch eine fahrbare Transportvorrichtung nach Anspruch 8 sowie durch ein Verfahren nach Anspruch 15. Vorteilhafte Ausgestaltungen finden sich in den Merkmalen der abhängigen Ansprüche.

Das erfindungsgemäße Führungssystem für medizinische Anlagen weist zumindest ein Bodenelement auf, welches sich entlang eines Weges der medizinischen Anlage erstreckt und in einem Bodenbereich des Weges angeordnet ist, und welches zur magnetischen Wechselwirkung derart ausgebildet ist, dass durch die magnetische Wechselwirkung des Bodenelements mit einem magnetischen Führungselement, das an einer fahrbaren Transportvorrichtung angeordnet ist, eine magnetische Anziehungskraft erzeugbar ist, wodurch eine Führung der fahrbaren Transportvorrichtung entlang des Bodenelements während eines Vorschubs der fahrbaren Transportvorrichtung entlang des Weges durch die medizinische Anlage erzielbar ist. Das Bodenelement kann aus Eisenblech und/oder ferromagnetischem Stahl gefertigt sein.

Das Führungssystem ermöglicht es, mit einer entsprechend ausgebildeten fahrbaren Transportvorrichtung unter Ausnutzung der magnetischen Anziehungskraft die fahrbare Transportvorrichtung auf dem Weg zu führen. Die magnetische Wechselwirkung bewirkt also eine magnetische Anziehungskraft zwischen dem Bodenelement und dem magnetischen Führungselement. Diese magnetische Anziehungskraft bewirkt direkt eine rückstellende Kraft auf die fahrbare Transportvorrichtung. Die magnetische Anziehungskraft übt also direkt eine Kraft auf die fahrbare Transportvorrichtung aus, sodass die Transportvorrichtung bei Vorschub zum dem Sollweg, der durch das Bodenelement charakterisiert ist, gezogen wird. Sollte die fahrbare Transportvorrichtung ausscheren und vom Weg etwas abweichen, bewirkt die magnetische Anziehungskraft, dass die fahrbare Transportvorrichtung wieder seitlich zu dem Weg zurückgezogen wird. Im Nahfeld des Bodenelementes, z.B. einer Schiene, ist diese Rückstellkraft umso größer, je größer die Abweichung von dem Bodenelement, z.B. von der Mittelachse der Schiene, ist.

Das hier offenbarte Führungssystem benötigt lediglich Elemente, die miteinander in magnetische Wechselwirkung treten. Das System kann also vergleichsweise einfacher aufgebaut werden, ist wartungsarm und bewirkt trotzdem eine effektive und kontaktfreie Führung der fahrbaren Transportvorrichtung.

Zudem wird durch das Führungssystem vermieden, dass eine fahrbare Transportvorrichtung bei einem Vorschub entlang eines Weges z.B. an kurvenreichen Stellen von der Sollbahn ausschert und an eine Wand stößt. Derartige Kollisionen können insbesondere bei der Strahlentherapie ein Problem darstellen, insbesondere, wenn ein Patient bereits in einer stereotaktischen Fixierung liegt und keine Erschütterungen erleiden darf.

Im Vergleich zu einer Lösung, bei dem eine Steuerung der fahrbaren Transportvorrichtung über optische oder elektromagnetisch-induktive Sensoren und eine entsprechende elektronische Steuerung bewerkstelligt wird, bietet das Führungssystem eine Reihe von Vorteilen. Die aufwändige Sensor - und Steuertechnik ist kostenintensiv, und in einem gewissen Maße auch fehleranfällig. Zudem bringen die Steuerung und die Motorik, welche an der fahrbaren Transportvorrichtung angeordnet werden müssen, einen höheren Platzbedarf und ein höheres Gewicht mit sich. Weiterhin muss sichergestellt werden, dass die Energieversorgung der Steuerung gewährleistet ist, beispielsweise über einen Akku, dessen Ladezustand überwacht werden muss, oder über Kabel, welche erhebliche Nachteile bezüglich der Beweglichkeit darstellen. Dieselben Vorteile ergeben sich auch gegenüber einer aktiven Steuerung der fahrbaren Transportvorrichtung, welche zur korrekten Führung der fahrbaren Transportvorrichtung Induktionsschleifen verwendet, die auf dem Boden aufgebracht sind.

Gegenüber einer Lösung, welche die Führung der fahrbaren Transportvorrichtung mithilfe einer Bodenschiene, die im mechanischen Kontakt mit der fahrbaren Transportvorrichtung steht, bewerkstelligt, bietet das erfindungsgemäße Führungssystem den Vorteil einer magnetischen und damit berührungslosen Führung. Hierdurch kann der Sicherheit einer medizinischen Anlage erhöht werden, da mechanische Schienensysteme gefährlich Stolperquellen oder Bodenunebenheiten darstellen und auch aus hygienischer Sicht problematisch zu reinigen sind, das hier vorgestellte Führungssystem aber einen vollkommen glatten Boden ermöglicht.

In einer vorteilhaften Ausführungsform ist das Bodenelement zumindest abschnittsweise als länglicher, schienenförmiger Körper aus einem ferromagnetischen Material ausgebildet. Insbesondere kann der längliche, schienenförmige Körper zumindest abschnittsweise Eisenblech und/oder ferromagnetischen Stahl umfassen oder aus Eisenblech und/oder aus ferromagnetischen Stahl bestehen.

Die Verwendung eines ferromagnetischen Materials bietet eine einfache Möglichkeit, eine magnetische Wechselwirkung mit einem magnetischen Führungselement der fahrbaren Transportvorrichtung zu gewährleisten. Ein Eisenblech und/oder der ferromagnetische Stahl kann einige Zentimeter breit sein (typischerweise zwischen 5 und 15 cm) und einige Millimeter Dicke aufweisen (typischerweise zwischen 2 und 20 mm).

Das Bodenelement, z.B. der längliche, schienenförmige Körper, kann sich am Beginn des Weges und/oder am Ende des Weges verjüngen. Die Verjüngung bewirkt, dass die magnetische Wechselwirkung mit der fahrbaren Transportvorrichtung am Ende schwächer wird. Dies ermöglicht ein ruckfreies Einfahren des Wagens auf die Schiene.

In einer vorteilhaften Ausgestaltung ist das Bodenelement zumindest abschnittsweise als ein Magnetfeld erzeugendes Element, insbesondere als Permanentmagnet, ausgebildet. Beispielsweise kann ein kurviger Abschnitt derart ausgebildet werden. Auf diese Weise kann die magnetische Wirkung verstärkt werden. Der oder die Magnete, die in diesem Abschnitt eingesetzt werden, sind entgegengesetzt dem Führungselement der fahrbaren Transportvorrichtung gepolt, damit eine anziehende Wirkung entsteht.

Vorzugsweise ist das Bodenelement im Boden eingelassen. Beispielsweise kann das Bodenelement mit dem Bodenbelag verpflegt oder eingearbeitet werden, so dass sich insgesamt eine glatte Fläche ergibt, also keine Ränder, Rillen oder Kanten vorhanden sind.

Die erfindungsgemäße medizinische Anlage weist ein derartiges Führungssystem auf. Insbesondere kann die medizinische Anlage als Partikeltherapieanlage ausgebildet sein. In diesem Fall umfasst die Partikeltherapieanlage zumindest einem Behandlungs- oder Diagnoseraum und einen Vorbereitungsraum, in welchem ein Patient für eine nachfolgende Diagnose oder Behandlung vorbereitet wird. Das Führungssystem ist entlang des Weges zumindest zwischen kurvigen Teilstrecken zwischen dem Vorbereitungsraum und dem Behandlungs- bzw. Diagnoseraum angeordnet. Auf diese Weise vereinfacht sich der Workflow erheblich.

Die erfindungsgemäße fahrbare Transportvorrichtung, insbesondere Patiententransportvorrichtung, weist ein magnetisches Führungselement auf, welches ausgebildet ist, mit einem Bodenelement, das in einem Bodenbereich angeordnet ist und das sich entlang eines Weges erstreckt, in magnetische Wechselwirkung zu treten, derart, dass durch die magnetische Wechselwirkung eine magnetische Anziehungskraft erzeugbar ist, wodurch eine seitliche Führung der fahrbaren Transportvorrichtung auf dem Weg während eines Vorschubs der fahrbaren Transportvorrichtung entlang des Weges erzielbar ist.

Bevorzugterweise ist das magnetische Führungselement als Permanentmagnet ausgebildet. Dies erlaubt eine Gewichts- und Platz sparende Bauweise der fahrbaren Transportvorrichtung. Der Permanentmagnet kann beispielsweise als Neodym-Eisen-Bor-Magnet (NdFeB) ausgebildet sein, da derartige Permanenzmagnete äußerst stark sind, eine hohe Anziehungskraft gewährleisten und damit eine ausreichend starke Führung. Bei einem Magnet-Durchmesser von 125 mm kann beispielsweise eine Haftkraft von 130 kg erreicht werden. Typische Maße für einen derartigen Magneten liegen zwischen 100 und 200 mm Durchmesser und 10 bis 30 mm Dicke. NdFeB-Magnete halten ihre Magnetisierung über einen großen Zeitraum, beispielsweise über 10 Jahre oder länger.

In vorteilhafter Weise ist der Permanentmagnet mit einem Kunststoffüberzug, der mehrere mm Dicke aufweisen kann, versehen. Dies verhindert eine direkte Berührung oder "Aufeinanderklatschen" des Magneten mit dem Bodenelement und ist besonders dann relevant, wenn der Wagen gefedert ist und somit kein starrer Abstand zwischen dem Permanentmagnet und dem Bodenelement gegeben ist.

Das magnetische Führungselement ist derart angeordnet, dass sich das magnetische Führungselement lediglich einen geringen Abstand zum Boden aufweist, üblicherweise weniger als 10 cm, insbesondere weniger als 5 cm und in besonders vorteilhafter Weise zwischen 2 und 20 mm. Auf diese Weise ist eine hohe Anziehungskraft gewährleistet.

In einer vorteilhaften Ausführungsform ist das magnetische Führungselement in der vorderen Hälfte der Transportvorrichtung angeordnet, wobei die vordere Hälfte bezüglich der Standardschubrichtung - d.h. derjenigen Schubrichtung, die bei standardgemäßen Gebrauch bevorzugt gefahren wird - definiert wird. Das magnetische Führungselement kann z.B. derart angeordnet werden, dass es in Schubrichtung vor dem vordersten Rad angeordnet ist. Die Transportvorrichtung kann eine Vorderachse aufweisen, die durch die Position des vordersten Rades gekennzeichnet ist. Das Führungselement kann im Bereich der Vorderachse, d.h. auf gleicher Position wie die Vorderachse oder ein Stück davor oder dahinter - bezogen auf die Schubrichtung -, angeordnet sein. Die genaue Wahl der Position des Führungselements kann je nach Größe, Achsenkonstruktion, Gewichtsverteilung und/oder Kurvenbeschaffenheit gewählt werden.

Das magnetische Führungselement liegt bevorzugterweise auf der Längs-Mittelachse der Transportvorrichtung.

In einer vorteilhaften Weiterbildung ist ein weiteres magnetisches Führungselement vorhanden, das in der hinteren Hälfte der Transportvorrichtung angeordnet ist, wobei die hintere dadurch gekennzeichnet ist, dass sie in Standardschubrichtung hinten liegt. Das weitere magnetische Führungselement kann z.B. derart angeordnet werden, dass es sich hinter dem hintersten Rad befindet. Die Transportvorrichtung kann eine Hinterachse aufweisen, die durch die Position des hintersten Rades gekennzeichnet ist. Das weitere Führungselement kann im Bereich der Hinterachse, d.h. auf gleicher Position wie die Hinterachse oder ein Stück davor oder dahinter - bezogen auf die Schubrichtung -, angeordnet sein. Eine derartige Anordnung ist besonders dann hilfreich, wenn der Wagen auch rückwärts geschoben werden soll. Aber auch bei einem Schub in Vorwärtsrichtung erfolgt eine verbesserte und definierte Lenkung entlang des Weges.

In einer weiteren Ausführungsform ist das Rad über einen Lenkmechanismus mit dem magnetischen Führungselement verbunden. Insbesondere die auf das magnetische Führungselement ausgeübte magnetische Anziehungskraft ist über den Lenkmechanismus auf das Rad übertragbar. Hierdurch kann die Radstellung zumindest teilweise durch die magnetische Anziehungskraft beeinflusst werden, derart, dass die Radstellung ein Lenken der fahrbaren Transportvorrichtung entlang des Weges bewirkt und damit einen zusätzlichen Effekt für die Rückführung auf die Sollbahn bewirkt.

Beim erfindungsgemäßen Verfahren zum Lenken einer fahrbaren Transportvorrichtung entlang eines Weges in einer medizinischen Anlage, in welcher entlang des Weges ein Führungssystem mit einem Bodenelement angeordnet ist, tritt das Bodenelement derart mit einem magnetischen Führungselement der fahrbaren Transportvorrichtung in magnetische Wechselwirkung, dass die fahrbare Transportvorrichtung beim Vorschub über eine magnetische Anziehungskraft zwischen dem magnetischen Führungselement und dem Bodenelement entlang des Bodenelements gelenkt wird.

Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein. Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:
- Fig. 1: eine Aufsicht auf einen Bestrahlungsraum einer Partikeltherapieanlage mit einem Zugangsweg, entlang dessen ein Führungssystem angeordnet ist.
- Fig. 2: eine vergrößerte Darstellung des mit II gekennzeichneten Bereichs aus Fig. 1,
- Fig. 3: eine vergrößerte Darstellung des mit III gekennzeichneten Bereichs aus Fig. 1,
- Fig. 4: eine Seitenansicht des mit IV gekennzeichneten Bereichs aus Fig. 3,
- Fig. 5: eine Seitenansicht einer als fahrbare Patientenliege ausgebildeten Patiententransportvorrichtung,
- Fig. 6: eine Aufsicht auf die fahrbare Patientenliege,
- Fig. 7: eine Frontalansicht der fahrbaren Patientenliege,
- Fig. 8: eine vergrößerte Darstellung des mit VIII gekennzeichneten Bereichs aus Fig. 7,
- Fig. 9: eine der Fig. 8 entsprechende Darstellung mit einem seitlichen Versatz des Magneten gegenüber der Magnetschiene,
- Fig. 10: eine Seitenansicht einer weiteren Ausführungsform einer fahrbaren Patientenliege,
- Fig. 11: eine Seitenansicht einer weiteren Ausführungsform einer fahrbaren Patientenliege mit einem Lenkmechanismus, und

- Fig. 12: eine Aufsicht auf die Patientenliege gemäß Fig. 11,
- Fig. 13: eine Seitenansicht einer im Vergleich zu Fig. 5 leicht modifizierten fahrbaren Patientenliege,
- Fig. 14: eine Ausführungsform des Aufbaus eines magnetischen Führungselementes mit zwei entgegengesetzt gepolten Permanentmagneten.

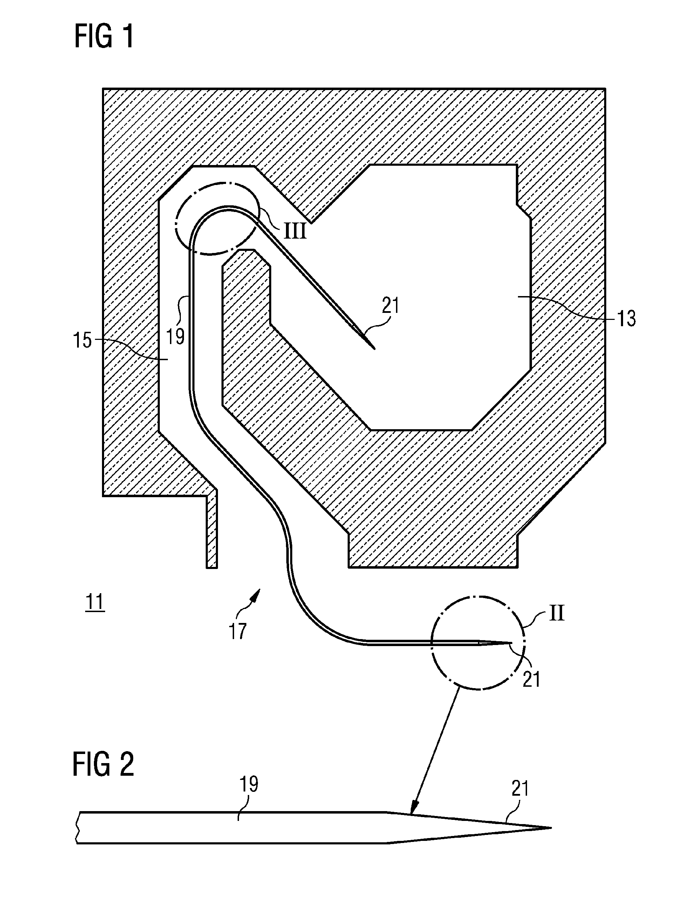

Fig. 1 zeigt einen Ausschnitt aus einer typischen Partikeltherapieanlage 11 mit einem Behandlungsraum 13. Aus strahlenschutztechnischen Gründen wird der Behandlungsraum 13 nur über einen labyrinthartigen Zutrittsweg 15 betreten. Entlang dieses Weges 15 muss ein Patient auf einer Patientenliege gefahren werden, um in den Behandlungsraum 13 zu gelangen. Entlang dieses Weges 15 ist ein Führungssystem 17 angeordnet, mit dessen Hilfe das Fahren der Patientenliege entlang des Weges 15 unterstützt wird, wie es anhand der nachfolgenden Figuren näher beschrieben wird.

Das Führungssystem umfasst ein schienenartig ausgebildetes Bodenelement 19, das in magnetische Wechselwirkung mit einer Patientenliege tritt. In den Endbereichen 21, d.h. beispielsweise in einem Vorbereitungsraum oder am Beginn des labyrinthartigen Weges 15 und/oder im Behandlungsraum 13 verjüngt sich das Bodenelement 19.

Dies ist deutlicher in Fig. 2 erkennbar, die den mit II gekennzeichneten Bereich vergrößert darstellt. Die Verjüngung des Bodenelements 19 in den Endbereichen 21 bewirkt, dass die magnetische Wechselwirkung mit dem Bodenelement 19 an dieser Stelle geringer ist, wodurch es möglich wird, eine Patientenliege weit gehend ruckfrei und sanft in das Führungssystem 17 einzufahren.

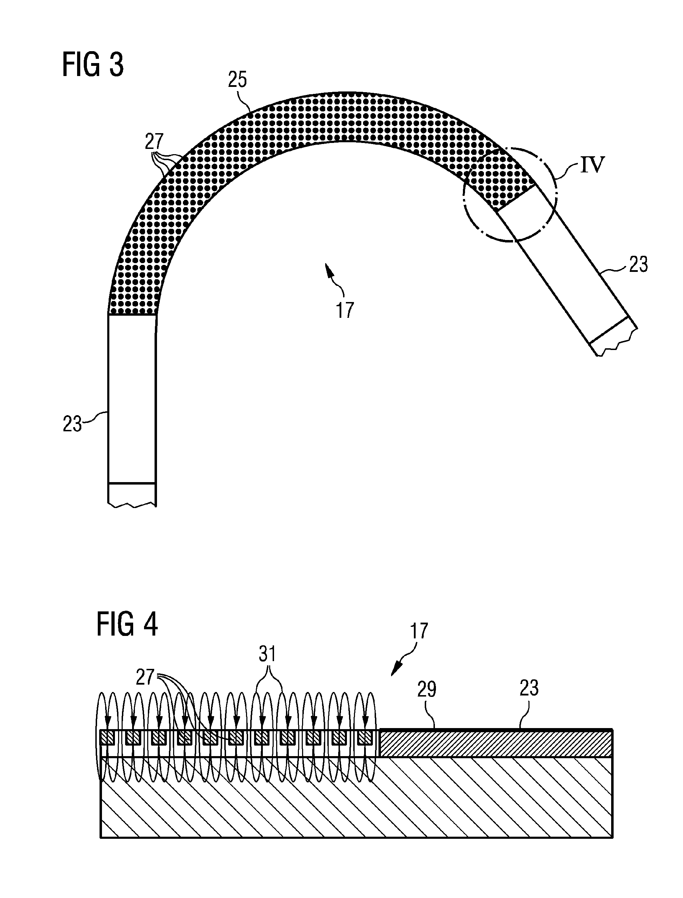

Fig. 3 zeigt eine vergrößerte Darstellung des mit III gekennzeichneten Bereichs aus Fig. 1. Es ist zu erkennen, dass das Bodenelement 19 abschnittsweise unterschiedlich aufgebaut sein kann. In Abschnitten, die im Wesentlichen gerade verlaufen, kann das Bodenelement ein längliches Eisenblech oder ferromagnetisches Stahlblech 23 umfassen. In anderen Abschnitten kann das Bodenelement 19 einer Vielzahl kleiner Permanentmagnete 27 umfassen, beispielsweise in stark gekrümmten Abschnitten 25. Dadurch, dass viele kleine Permanenzmagnete 27 verwendet werden, welche typischerweise eine Größe von 5 bis 20 mm aufweisen können, ist eine kontinuierliche Fahrt der Patientenliege möglich, so dass eine Ruckeln durch die Anziehung von den einzelnen Bodenmagneten weitgehend vermieden wird.

Fig. 4 zeigt eine Seitenansicht des mit IV gekennzeichneten Bereichs aus Fig. 3. Sowohl das Eisenblech 23 als auch die Vielzahl kleiner Permanenzmagnete 19 sind in den Boden eingelassen und mit einem Überzug 29 versehen, so dass eine glatte Bodenfläche entsteht. Weiterhin eingezeichnet sind die Magnetfeldlinien 31, die das Magnetfeld der kleinen Permanenzmagnete 27 symbolisieren. Das Magnetfeld der Permanentmagnete 27 ist derart orientiert, dass sich durch Wechselwirkung mit einem magnetischen Führungselement der Patientenliege eine anziehende Kraft ergibt.

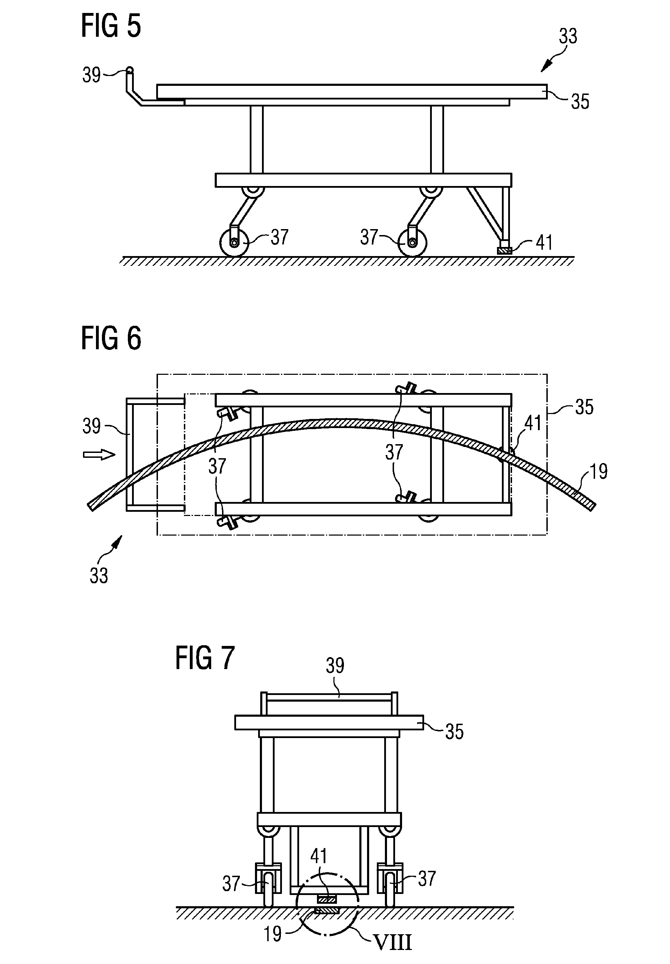

Fig. 5 zeigt eine Seitenansicht einer fahrbaren Patientenliege 33. Der Patient (nicht dargestellt) wird auf einer Tischplatte 35 positioniert. Eine Patientenliege 33 weist üblicherweise vier Räder 37 auf, teilweise auch fünf oder sechs Räder, sodass die Patientenliege 33 fahrbar ist. Ein Anwender (nicht dargestellt) schiebt üblicherweise die Patientenliege 33 vor sich her. Die Patientenliege weist durch ihren Aufbau eine bevorzugte Schubrichtung auf, in die die Patientenliege bei standardgemäßem Gebrauch bevorzugt geschoben wird. Die Patientenliege 33 kann hierfür einen Griff aufweisen 39. Üblicherweise sind zwei der Räder 37 oder alle vier Räder 37 beweglich, so dass mit der Patientenliege 33 auch kurvige Strecken gefahren werden können.

Im vorderen Bereich - gesehen aus Sicht der Richtung gesehen, in der die Patientenliege bei standardgemäßem Gebrauch vorzugsweise geschoben wird - der Patientenliege 33 ist ein Permanentmagnet 41 angeordnet, der vorzugsweise aus einem Material besteht, das Neodym-Eisen-Bor umfasst. Wie hier dargestellt kann der Permanentmagnet 41 vor der Vorderachse (der Achse der vorderen Räder) angeordnet sein, aber auch auf Höhe der Vorderachse oder gar etwas hinter der Vorderachse. Der Permanentmagnet 41 weist einen geringen Abstand vom Boden auf, typischerweise zwischen 1 mm und 10 mm.

Fig. 6 zeigt eine Aufsicht auf die Patientenliege 33. In dieser Darstellung ist das Bodenelement 19 zu sehen, dass in magnetische Wechselwirkung mit dem Permanentmagneten 31 der Patientenliege 33 tritt. Wenn die Patientenliege 33 in Pfeilrichtung geschoben wird, bewirkt die magnetische Anziehungskraft zwischen dem Permanentmagneten 41 und dem Bodenelement 19, dass die Patientenliege 33 entlang des Bodenelements 19 geführt wird.

Fig. 7 zeigt eine Frontansicht der Patientenliege 33. In dieser Ansicht ist zu sehen, dass das Bodenelement 19 im Boden eingelassen ist, wodurch eine glatte Bodenoberfläche ohne Rillen und Kanten entsteht.

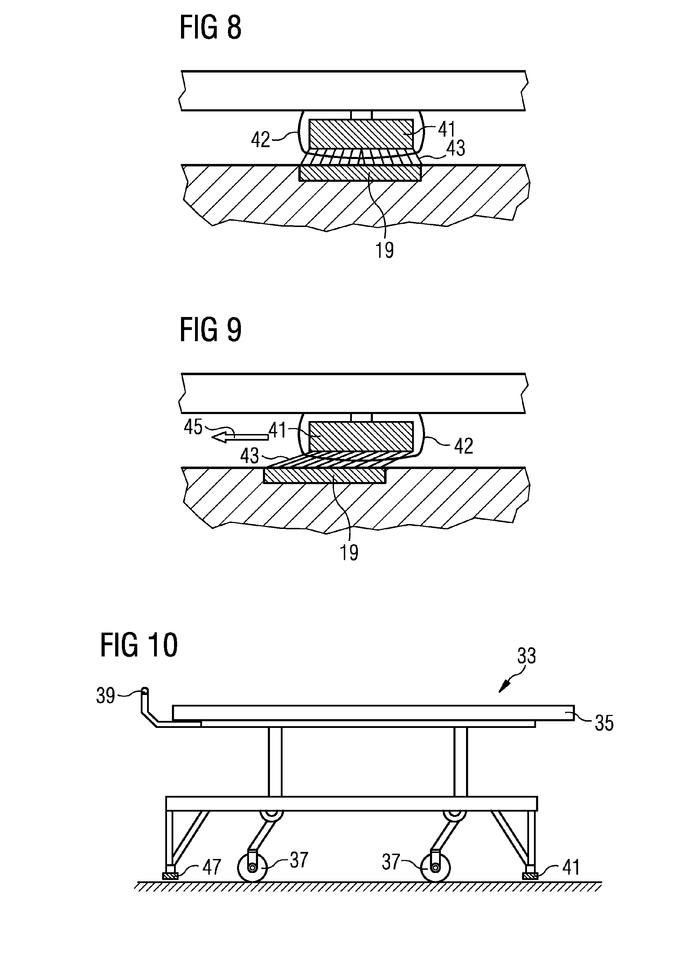

Fig. 8 zeigt eine vergrößerte Darstellung des mit VIII gekennzeichneten Bereichs aus Fig. 7. Zu sehen ist die magnetische Wechselwirkung zwischen dem Permanentmagneten 41 und dem Bodenelement 19, symbolisiert durch die Magnetfeldlinien 43. Der Permanentmagnet 41 kann mit einem Kunststoffüberzug 42 versehen sein.

Fig. 9 erläutert die Situation, die auftritt, wenn sich beim Vorschub die Patientenliege 33 von dem Bodenelement 19 entfernt. Durch die magnetische Anziehungskraft 45 wird der Permanentmagnet 41 zu dem Bodenelement 19 hingezogen, so dass die Patientenliege 33 automatisch entlang des Bodenelements 19 geführt wird. Dies geschieht mit sehr geringem zusätzlichem menschlichem Kraftaufwand, weil die Kräfte nicht bremsend wirken, sondern durch die Räder aufgenommen werden und höchstens durch die höhere vertikale Auflagekraft der Rollen eine geringfügig höhere Rollreibung erzeugen.

Fig. 10 zeigt eine Patientenliege 33, die gegenüber der in Fig. 5 gezeigten Patientenliege 33 ein weiteres Führungselement 47 aufweist, welches im hinteren Bereich der Patientenliege 33 angeordnet ist. Dieses weitere Führungselement 47 ist insbesondere dann vorteilhaft, wenn die Patientenliege 33 in entgegengesetzter Richtung gefahren wird.

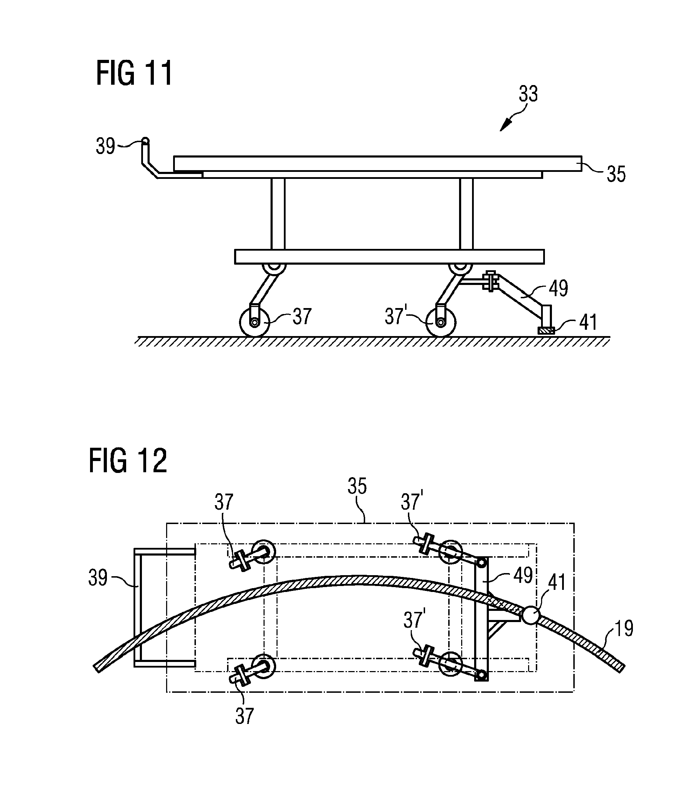

Fig. 11 zeigt eine Patientenliege 33, die gegenüber der in Fig. 5 gezeigten Patientenliege den Unterschied aufweist, dass der Permanentmagnet 41 über eine Lenkmechanismus 49 mit den vorderen beweglichen Rädern 37' gekoppelt ist.

Anhand von Fig. 12 wird die Wirkung des Lenkmechanismus 49 näher erläutert. Durch den Lenkmechanismus 49 werden die beiden Vorderräder 37 ' parallel geführt. Der Lenkmechanismus 49 ist derart mit dem magnetischen Führungselement - in diesem Falle mit dem Permanentmagneten 41 - gekoppelt, dass der Lenkmechanismus 49 die beiden Vorderräder entsprechend der Kraft auf den Permanentmagneten 41, durch welche der Permanentmagnet 41 zum Bodenelement 19 hingezogen wird, in Richtung auf die Sollkurve lenkt.

Falls die Patientenliege 33 also von ihrer Sollkurve abkommt
- falls sich der Permanentmagnet 41 also nicht mehr zentriert über dem Bodenelement 19 befindet - wirkt auf den Permanentmagneten 41 ein horizontales Moment zum Bodenelement 19 hin. Das wesentlich größere vertikale Moment spielt in diesem Fall keine Rolle. Das horizontale Moment wird über einen Hebelmechanismus an die Lenkung der Vorderräder 37' übertragen, so dass die Patientenliege 33 auf die Sollkurve zurückgeführt wird.

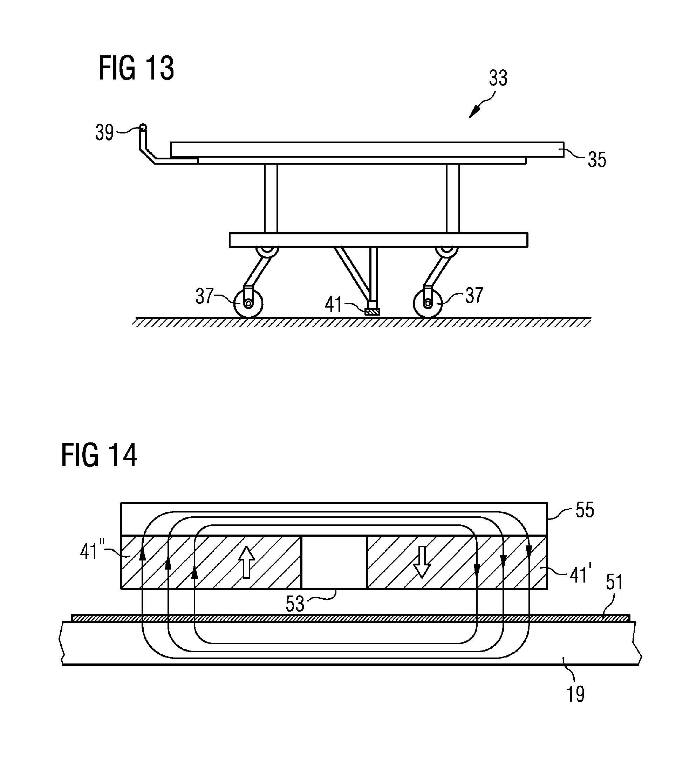

Fig. 13 zeigt eine Seitenansicht einer im Vergleich zu Fig. 5 leicht modifizierten fahrbaren Patientenliege 33. Hier ist der Permanentmagnet 41 zwischen den Vorderrädern und den Hinterrädern angeordnet.

Fig. 14 zeigt eine besondere Ausführung des Führungselementes, bei dem der eine Permanentmagnet durch zwei nebeneinander angeordnete entgegengesetzt gepolte Permanentmagnete 41', 41'' ersetzt wird. Die entgegengesetzte Polung ist durch die dicken Pfeile symbolisiert. Die Permanentmagnete 41', 41'' können aus NdFeB sein und eine Quaderform mit den Abmessungen 50x75x10 mm aufweisen. Der Abstand zwischen Bodenelement 19 und den Permanentmagneten 41', 41'' kann 10 mm betragen. Über dem Bodenelement 19, z.B. einer 8 mm dicken Stahlschiene, kann ein Bodenbelag 51, z.B. aus Linoleum, angeordnet sein.

Zwischen den beiden Magneten kann ein schmaler Abstandshalter 53 z.B. aus Kunststoff eingebaut sein, der einen Abstand von 20 mm zwischen den beiden Permanentmagneten hält.

Über den beiden Permanentmagneten liegt ein gemeinsames Joch 55 aus ferromagnetischem Material (also z.B. ein dickes Stahlblech).

Der Vorteil dieser Anordnung liegt darin, dass die Magnetfeldlinien in dieser Anordnung geschlossen durch den Komplex aus Bodenelement 19, erster Permanentmagnet 41', Joch 55, zweiter Permanentmagnet 41'' verlaufen. Damit wird eine besonders effiziente Wirkung erzielt: es wird also eine hohe Anziehung bei geringerer Größe bzw. geringerem Gewicht der Magnete erreicht und die magnetischen Streufelder - also unnütze Feldlinien außerhalb des System - reduziert.

### Bezugszeichenliste

- 11: Partikeltherapieanlage
- 13: Behandlungsraum
- 15: Zutrittsweg
- 17: Führungssystem
- 19: Bodenelement
- 21: Endbereich

- 23: Eisenblech, Strahlblech
- 25: gekrümmter Abschnitt
- 27: Permanentmagnete
- 29: Überzug
- 31,: 43 Magnetfeldlinien
- 33: Patientenliege
- 35: Tischplatte
- 37, 37': Rad
- 39: Griff
- 41: Permanentmagnet
- 45: magnetische Anziehungskraft
- 47: weiteres Führungselement
- 49: Lenkmechanismus
- 51: Bodenbelag
- 53: Abstandshalter
- 55: Joch

## Patentansprüche

1. Führungssystem für medizinische Anlagen, insbesondere Kliniken, aufweisend
zumindest ein Bodenelement (19), welches sich entlang eines Weges (15) der medizinischen Anlage (11) erstreckt und in einem Bodenbereich des Weges (15) angeordnet ist, und welches zur magnetischen Wechselwirkung derart ausgebildet ist,
dass durch die magnetische Wechselwirkung des Bodenelements (19) mit einem magnetischen Führungselement (41, 41', 41''), das an einer fahrbaren Transportvorrichtung (33) angeordnet ist, eine magnetische Anziehungskraft erzeugbar ist, wodurch eine Führung der fahrbaren Transportvorrichtung (33) entlang des Bodenelements (19) während eines Vorschubs der fahrbaren Transportvorrichtung (33) entlang des Weges (15) durch die medizinische Anlage (11) erzielbar ist.

2. Führungssystem nach Anspruch 1,
wobei das Bodenelement (19) zumindest abschnittsweise als länglicher, schienenförmiger Körper aus einem ferromagnetischen Material ausgebildet ist und insbesondere Eisenblech oder ferromagnetischen Stahl (23) umfasst.

3. Führungssystem nach Anspruch 1 oder 2,
wobei sich das Bodenelement (19) an einem Beginn und/oder am Ende des Weges (15) verjüngt.

4. Führungssystem nach einem der Ansprüche 1 bis 3,
wobei das Bodenelement (19) zumindest abschnittsweise eine ein Magnetfeld erzeugende Komponente (27), insbesondere zumindest einen Permanentmagneten, umfasst.

5. Führungssystem nach einem der Ansprüche 1 bis 4,
wobei das Bodenelement (19) im Boden eingelassen ist und insbesondere mit dem Boden eine glatte Fläche bildet.

6. Medizinische Anlage aufweisend zumindest einen Patiententransportkorridor mit einem Führungssystem (17) nach einem der Ansprüche 1 bis 5.

7. Medizinische Anlage nach Anspruch 6, welche als Partikeltherapieanlage (11) ausgebildet ist, welche zumindest einen Behandlungs- oder Diagnoseraum (13) umfasst, wobei das Führungssystem (17) entlang eines Weges (15) in den Behandlungs- bzw. Diagnoseraum (13) angeordnet ist.

8. Fahrbare Transportvorrichtung, insbesondere Patiententransportvorrichtung,
aufweisend ein magnetisches Führungselement (41, 41', 41'', 47), welches ausgebildet ist, mit einem Bodenelement (19), das in einem Bodenbereich angeordnet ist und das sich entlang eines Weges (15) erstreckt, in magnetische Wechselwirkung zu treten, derart, dass durch die magnetische Wechselwirkung eine magnetische Anziehungskraft erzeugbar ist, wodurch eine Führung der fahrbaren Transportvorrichtung(33) auf dem Weg (15) während eines Vorschubs der fahrbaren Transportvorrichtung(33) entlang des Weges (15) erzielbar ist.

9. Fahrbare Transportvorrichtung nach Anspruch 8, wobei das magnetische Führungselement als Permanentmagnet (41, 41', 41'', 47), insbesondere als Neodym-Eisen-Bor-Magnet, ausgebildet ist.

10. Fahrbare Transportvorrichtung nach Anspruch 8 oder 9, wobei
das magnetische Führungselement (41, 41', 41'', 47) mit einem Kunststoffüberzug (42) versehen ist.

11. Fahrbare Transportvorrichtung nach einem der Ansprüche 8 bis 10, wobei
das magnetische Führungselement (41, 41', 41'', 47) einen Abstand zum Boden von weniger als 10 cm aufweist.

12. Fahrbare Transportvorrichtung nach einem der Ansprüche 8 bis 11, wobei
die fahrbare Transportvorrichtung (33) eine in Schubrichtung vorne gelegene vordere Hälfte aufweist, , und wobei das magnetische Führungselement (41) in der vorderen Hälfte (37, 37') angeordnet ist.

13. Fahrbare Transportvorrichtung nach Anspruch 12, wobei die fahrbare Transportvorrichtung (33) ein weiteres magnetisches Führungselement (43) umfasst, welches in der in Schubrichtung hinten gelegenen hinteren Hälfte angeordnet ist.

14. Fahrbare Transportvorrichtung nach Anspruch 12 oder 13, wobei
die fahrbare Transportvorrichtung zumindest ein Rad (37') aufweist, das über einen Lenkmechanismus (49) mit dem magnetischen Führungselement (41, 41', 41'', 47) verbunden ist, wodurch insbesondere die auf das magnetische Führungselement (41) ausgeübte magnetische Anziehungskraft das Rad (37') übertragbar ist.

15. Fahrbare Transportvorrichtung nach einem der Ansprüche 8 bis 14, wobei das Führungselement (41) zwei entgegengesetzt gepolte, voneinander beabstandet angeordnete Permanentmagnete (41', 41") umfasst.

16. Verfahren zum Lenken einer fahrbaren Transportvorrichtung (33) entlang eines Weges (15) in einer medizinischen Anlage (11), wobei entlang des Weges (15) ein Führungssystem (17) mit einem Bodenelement (19) angeordnet ist, welches Bodenelement (19) derart mit einem magnetischen Führungselement (41, 41', 41'', 47) der fahrbaren Transportvorrichtung(33) in magnetische Wechselwirkung tritt, dass die fahrbare Transportvorrichtung (33) beim Vorschub über eine magnetische Anziehungskraft zwischen dem magnetischen Führungselement (41, 41', 41'', 47) und dem Bodenelement (17) entlang des Weges (15) gelenkt wird.
